# EUROPEAN PATENT APPLICATION

(11) **EP 1 285 590 A1**
(43) Date of publication of application: **26.02.2003**
(21) Application number: 01119155.8
(22) Date of filing: 08.08.2001
(51) Int. Cl.: A23L 1/30, A61K 31/19

(54) **Lipid blends**

(71) Applicant: Société des Produits Nestlé S.A., 1800 Vevey (CH)
(72) Inventor: Decombaz, Jacques, 1806 St-Legier (CH); Mace, Catherine, 1095 Lutry (CH)
(74) Representative: Becker Kurig Straus

(57) **Abstract**

The present invention pertains to nutritional compositions containing specific blends of dietary lipids adapted to specifically influence the level of intramyocellular lipids in muscle tissue towards either higher concentrations or lower concentrations.

## Description

The present invention pertains to nutritional compositions containing specific blends of dietary lipids adapted to specifically influence the level of intramyocellular lipids in muscle tissue towards either higher concentrations or lower concentrations.

The major energy sources for mammalian muscle cells are carbohydrates, in particular glycogen, and fat.

Glycogen, a macromolecule comprised of up to 120.000 glucose monomers is stored in discrete granules in the cytoplasm of muscle and liver cells, which granules also contain the enzymes required for the synthesis or degradation of said polymer. Degradation of glycogen in muscle and liver cells is effected upon an external signal, such as a high energy requirement by muscle cells or a low blood glucose level.

In the body glycogen is primarily used for quickly providing energy, since glucose may also be degraded anaerobically. Moreover, apart from providing a constant glucose level in the blood, the concentration of glycogen in the muscles has been shown to be one of the major determinants for endurance capacity. In consequence, for athletes it is important to ingest adequate amounts of carbohydrates before sportive activity to increase endurance.

Fat, apart from providing essential fatty acids and a solvent system for vitamins, also represents a major fuel for mammalian daily physical activities. It is available for oxidation in muscle cells both from extramuscular sources, represented by circulating lipids, and intramuscular sources.

The intramuscular source is essentially comprised by two distinct lipid compartments. One source is constituted by adipocytes, present in-between muscle fibers and designated extra-myocellular lipids (EMCL). Another compartment for lipid storage is represented by discrete lipid droplets in contact with muscle mitochondria, which lipid source is termed intramyocellular lipids (IMCL).

Depending on the workload, muscle energy metabolism uses different proportions of carbohydrates and lipids. In the recent past, research has shown that an unexpectedly high proportion of the lipid energy during endurance exercise was being derived from muscle triglycerides.

To this end, it has been observed that prolonged sustained exercise enlarges muscle triglyceride stores (Morgan et al., Am. J. Physiol. **216** (1969), 82-86) as well as fatty acid uptake and oxidation (Turcotte et al., Am. J. Physiol. **262** (1992), E791-E799), which fact is deemed to be one of the reasons for sparing glycogen during submaximal periods of an exercise and eventually add to an increased physical performance.

On the other hand, the provision of high carbohydrate food material, which is known to be optimal for glycogen loading before activity, might also lead to a reduction in muscle fat stores. In addition, prolonged sustained exercise is considered to essentially lead to a depletion of IMCL (Oberholzer et al., Schweiz Z. Sportmed. **24** (1976), 71-98). It is, therefore, presently deemed that exercise conditions may exist that limit the exercise capacity simply due to a reduced availability of locally stored lipids.

Consequently, there is a need in the art for means to influence the intramyocellular lipid level in muscle cells, so as to provide an optimal balance between glycogen and IMCL as energy stores.

An object of the present invention resides therefore in providing such means.

This problem has been solved by a lipid blend containing particular lipids in an amount such that the level of intramyocellular lipids in muscle cells of the respective individuals is influenced.

### In the figures

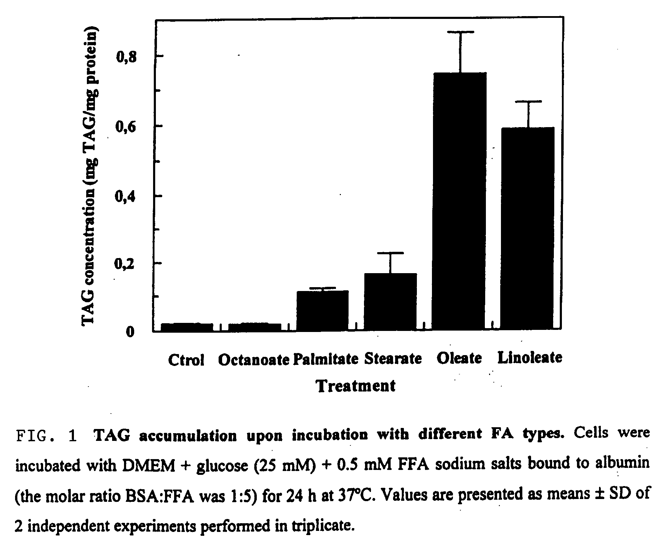

Fig. 1 shows the results of experiments, wherein muscular cells have been exposed to various fatty acids.

A lipid blend of the present invention, designed to promote IMCL storage, contains oleic acid in an amount of from 50-70%, n-6 linoleic acid in an amount of from 20-35%, n-6 linolenic acid or longer chain fatty acids of the n-6 family in an amount of from 15-25%, stearic and palmitic acids, both together in an amount of from 0-15%, and polyunsaturated fatty acids of the n-3 family in an amount of from 1-10%, each in form of glycerides and all based on the final fat content of the nutritional product.

Examples for polyunsaturated fatty acids to be utilized are n-3 linoleic, n-3 linolenic, eicosatrienoic (C20:2 n-6 cis), dihomo-gamma-linolenic, arachidonic, eicosapentaenoic (C20:5 n-3 cis) and docosahexaenoic (C22:6 n-3 cis) acids.

The major task of the stearic and palmitic acids in the present blend mainly resides in alleviating a mixing of the blend for the producer.

The lipid blend may be used as such, e.g. as an edible oil, but may likewise be included in a carbohydrate-containing food, with no less than 30%, preferably 30-70%, of the energy as fat. Insulinogenic proteins, such as from whey fractions or hydrolysates, which will provide 0-20% of energy, and additionally amino acids, such as leucine (0-5% of energy) and/or arginine (0-5% of energy), may also be included in the composition.

According to an alternative embodiment the present invention also provides a lipid blend designed to reduce the accumulation of IMCL in an individual. The subjective blend contains medium-chain triglycerides (C₆-C₁₂ fatty acids) in an amount of from 40-65%, and triglycerides with long-chain (≥ C₁₄ chain length) saturated fatty acids preferably esterified in positions 1 and/or 3 of the glycerol molecule in an amount of from 20 - 50 %, and monounsaturated or polyunsaturated fatty acids preferably esterified in position 2 of the same glycerol molecule where the long chain fatty acids are bound, in an amount of from 0-30%.

Examples for C₆-C₁₂ fatty acids are hexanoic, octanoic, decanoic and dodecanoic acids, and examples for long chain fatty acids are palmitic, palmitoleic, stearic and oleic acids, while examples for polyunsaturated fatty acids to be used in the present blend are linoleic, linolenic, eicosatrienoic (C20:2 n-6 cis), dihomo-gamma-linolenic, arachidonic and eicosapentaenoic (C20:5 n-3 cis) acids.

The role of the mono- or poly-unsaturated fatty acids is primarily to render the molecule more susceptible to hydrolysis in the gastro-intestinal tract.

The lipid blend may be used as such. Yet, compositions comprising such lipid mixtures as described above may also contain cocoa butter and/or palm kernel oil (as components of the medium-chain triglyceride fraction), and mono- and di- as well as other tri-glycerides, and triacetin (0-10% of the fat).

The final composition may also include minerals, in particular calcium or magnesium (0.5-2.0% per weight) as salts. This feature allows to render part of the fat indigestible, and thus unavailable for the body.

Other constituents may be added to the composition to either enhance or inhibit IMCL deposition, such as carnitine and creatine, or to improve the nutritional value, such a vitamins or other essential fatty acids, or to ensure appropriate shelf life, such as food grade antioxidants.

The final nutritional formulation may be in liquid as well as in solid food form. In principle, the lipid blends of the present invention may be used as such or be included in any food material, with the proviso that the fat intake of an individual is primarily effected by means of the lipidic blend of the present invention, to obtain the desired objective, namely providing an increased or decreased IMCL level. Examples for such food materials are milk, yogurt, curd, cheese, fermented milks, milk based fermented products, ice-creams, fermented cereal based products, milk based powders, infant formulae, energy bars, liquid foods, formulae for clinical enteral nutrition, energy drinks and pet food.

The specific lipid blends object of the invention may also be used the same way as an edible oil, as ingredients (such as in salad dressing or for baking cakes) or as frying oils.

It will be understood that also mixtures of the two lipidic blends are comprised by the present invention, which will result in the IMCL level to slightly increase or decrease or to stay constant. Lifestyle factors such as previous exercise, ordinary physical activity, caloric restriction or punctual caloric overfeeding may be used in combination with the lipid blends to reach the desired effect.

According to the present invention it is now possible to influence the accumulation of intramyocellular lipids (IMCL) towards either higher concentrations, or lower concentrations, using nutritional or food compositions containing the specific blends of dietary lipids and optionally other components, with the purpose to modulate functions. A larger accumulation of IMCL will eventually provide an extra source of local lipid energy for endurance athletes improving their physical performance. A reduced accumulation will contribute to improve metabolic control in e.g. sedentary individuals and individuals with a degree of glucose intolerance and insulin resistance, hence promote better health.

When ingested as a fraction of the daily food, a food composition of the present invention with appropriately designed lipid mixtures will promote IMCL storage. In the treatment designed to boost up IMCL, the invention provides the advantage that IMCL stores of endurance athletes can be predictably increased so as to maximize performance. Therefore, an advantage provided by the present invention resides in that endurance athletes may increase or decrease their IMCL level in a controlled manner during the dietary preparation for an event, or after an event, when wishing to maximize muscle energy stores as IMCL.

The basic concept of the present invention may be seen in understanding that, what is critical for performance, is filling a specific lipid fuel tank, not inducing chronic metabolic adaptation, it allows much shorter periods of fat feeding. This is an advantage because high fat feeding during continued training is perceived by athletes as particularly hard and tiring. The invention is used in addition to dietary strategies to promote muscle glycogen storage, so that the recovery or filling up (even overcompensation) of both intramuscular energy fuels essential for performance, IMCL and glycogen, is optimized. The carbohydrate component, contained in the composition according to the present invention, assists in channeling the dietary lipids towards storage rather than oxidation.

In the treatment designed to prevent IMCL accumulation, the invention provides the advantage that IMCL stores of sedentary persons can be predictably reduced so as to improve insulin sensitivity, thereby benefit their general health. One advantage of the present invention ensues from cross-sectional observations showing that insulin resistance is positively and independently correlated with IMCL in non-athletic individuals. The advantage of the particular lipid blends used to prevent IMCL finds support in observations that different fatty acids accumulate in vitro in muscle cells to varying extent, and is based on the evidence of the existence of a molecular link between an increased availability of saturated fatty acids and the establishment of insulin resistance in skeletal muscle. The use of medium-chain glycerides is supported by the observation that these lipids are oxidized faster and stored with a lesser efficiency in the body than long-chain glycerides. The inclusion of minerals in the composition is supported by observations that saturated long-chain fatty acids (especially when they are in position 1or 3 on the glycerol molecule) are saponified and excreted as calcium (or magnesium) salts undigested in the feces, with the effect that a fraction of them does not even reach the blood.

The invention will now be described by means of non limiting examples.

### Example 1

### Preparation of nutritional compositions for increasing or decreasing IMCL storage.

Human skeletal muscle cells were used as a model to test the effect that different fatty acids (as precursors) may have on the extent of muscle triglyceride storage.

Primary cultures were initiated from a bank of satellite cells of muscle biopsies obtained from patients free of muscle disease. Aneural muscle cultures were established in a monolayer, then they were grown in a DMEM-M-199 medium, 3:1, supplemented with 10% fetal bovine serum, 10µg/ml insulin, 2 mM glutamine, 25 ng/ml fibroblast growth factor, and 10 ng/ml epidermal growth factor. Immediately after myoblast fusion, cells were rinsed in Hank's balanced salt solution and a medium devoid of FGF, EGF, and glutamine was added. Muscle cultures were maintained in this medium for up to 4 weeks.

Cells were incubated with DMEM + glucose (25 mM) + 0.5 mM FFA salt bound to albumin (in independent experiments for each FFA), for 24 hrs at 37°C. As examples for the fatty acids used in blend 1 (promoting IMCL storage), oleate (C18:1 cis), linoleate (C18:2 cis/cis), n-6 linoleate and n-3 linoleate.

As examples for the fatty acids used in blend 2 (inhibiting IMCL storage), octanoate (C8:0), palmitate (C16:0) and stearate (C18:0).

After incubation, cell lipids were extracted according to the method of Bligh and Dyer (Can. J. Biochem Physiol **37**: 911-917, 1959). To determine the IMCL content, extracts were prepared by scraping cell monolayers in a buffer consisting of 50 mM Tris, 100 mM KCl, 20 mM KF, 0.5 mM EDTA and 0.05% Lubrol PX, pH 7.9, and they were sonicated three times for 5 seconds. Homogenates were centrifuged at 11'000 g for 15 min and the resulting supernatants were collected. Total triacylglycerol (i.e. IMCL) was measured with a Cobas-Bio autoanalyzer with a GPO-Trinder kit, with triolein resuspended in the extraction buffer as a standard. The intramyocellular nature of the triglycerides was evidenced by histochemical staining with Sudan III in 70% ethanol.

As it is shown in Figure 1, no accumulation of IMCL was observed in cells exposed to the medium-chain length FA octanoate (comparable to the FA acid free control medium), whereas significant accumulation was achieved after incubation with long-chain FA. The saturated FA palmitate and stearate induced a moderate synthesis, with no significant difference between them. In contrast, a notably higher synthesis was observed from unsaturated FA, oleate and linoleate, compared to saturated FA (between 2.5 and 4-fold higher).

This example demonstrates that muscle cells exposed to different fatty acids accumulate them to a variable extent, resulting in either higher or lower IMCL levels.

### Example 2

### Ability to modulate IMCL in man by diet

Using proton NMR spectrometry, the effect of the diet on IMCL storage in man was investigated. Six endurance-trained subjects were submitted to 2hrs exercise (designed to decrease IMCL stores in muscle) after which they followed a diet low in fat (15% of energy as lipids) for one and a half day.

On another occasion, they followed a diet rich in fat (55% of energy as lipids) after the exercise. More than 50% of the fatty acids in the high-fat were provided as a food product with a fatty acid profile selected to promote IMCL storage, in this case : oleic acid 59%, linoleic acid 26%, palmitic acid 5% and stearic acid 3%. The diets were isocaloric.

IMCL levels were measured before and after the diet in the *tibialis anterior* muscle of the right leg according to Boesch et al., Magn. Reson. Med (1997) 27: 484-493.

| IMCL content mmol/kg wet muscle | low-fat diet | high-fat diet |
|---|---|---|
| pre-diet | 2.53±1.13 | 2.53±1.55 |
| post-diet | 2.73±1.15 | 4.25±1.99 |

The change in IMCL levels after the low-fat diet was not different from zero. After the high-fat diet, IMCL levels increased 68% (P<0.001).

This example demonstrates that a person exposed to a product providing lipids with fatty acids selected to promote storage, accumulate IMCL.

## Claims

1. A lipid blend, designed to increase the intramyocellular lipid level in an individual,
**characterized in that** it contains
oleic acid of from 50-70%,
n-6 linoleic acid of from 20-35%,
n-6 linolenic acid or longer chain fatty acids of the n-6 family of from 15-25%,
stearic and palmitic acids, together in an amount of from 0-15%, and
polyunsaturated fatty acids of the n-3 family from 1-10%.

2. A lipid blend designed to decrease the accumulation of intramyocellular lipid in an individual, **characterized in that** it contains
medium-chain fatty acids in an amount of from 40-65%;
glycerides with long-chain saturated fatty acids of from 20 - 50%, and
monounsaturated or polyunsaturated fatty acids in an amount of from 0-30%.

3. A food product **characterized in that** it contains a lipid blend according to claim 1 or claim 2.

4. The food product according to claim 3, **characterized in that** it contains carbohydrates.

5. The food product according to claim 3 or 4, which further comprises insulinogenic proteins, and optionally additionally amino acids.

6. A food product **characterized in that** it contains a lipid blend according to claim 5.

7. A food product according to any of the claims 3 to 6, which is selected from milk, yogurt, curd, cheese, fermented milks, milk based fermented products, ice-creams, fermented cereal based products, milk based powders, infant formulae, energy bars, liquid foods, formulae for clinical enteral nutrition, energy drinks and pet food.

8. Use of a lipid blend according to claim 1 or claim 2 for the preparation of a food product

9. The use according to claim 8, wherein the food product is selected from milk, yogurt, curd, cheese, fermented milks, milk based fermented products, ice-creams, fermented cereal based products, milk based powders, infant formulae, energy bars, liquid foods, formulae for clinical enteral nutrition, energy drinks and pet food.

10. A process for the preparation of a food product designed to increase or decrease the intramyocellular lipid level in an individual, which comprises the steps of mixing a lipid blend according to claim 1 or 2 with an ingestable carrier.
